# EUROPEAN PATENT SPECIFICATION

(11) **EP 2 470 168 B1**
(45) Date of publication and mention of the grant of the patent: **31.01.2018**
(21) Application number: 10747777.0
(22) Date of filing: 24.08.2010
(51) Int. Cl.: A61K 31/122, A61P 25/28

(54) **METHODS FOR THE PREVENTION AND TREATMENT OF CEREBRAL ISCHEMIA**
VERFAHREN ZUR PRÄVENTION UND BEHANDLUNG VON ZEREBRALER ISCHÄMIE
PROCÉDÉS DE PRÉVENTION ET DE TRAITEMENT D UNE ISCHÉMIE CÉRÉBRALE

(30) Priority: 26.08.2009 US 275269 P
(43) Date of publication of application: 04.07.2012
(73) Proprietor: BioElectron Technology Corporation, Mountain View, CA 94043 (US)
(72) Inventor: MILLER, Guy, M., Mountain View CA 94043 (US); KHEIFETS, Viktoria, Mountain View CA 94043 (US)
(74) Representative: J A Kemp
(86) International application number: PCT/US2010/046503
(87) International publication number: WO 2011/025785

(56) References cited:
- WO-A1-2010/030607
- WO-A2-2007/100652
- WO-A2-2009/111543
- "Coenzyme-Q used to treat headaches, dizziness etc.. administered orally or parenterally" WPI WORLD PATENT INFORMATION DERWENT, vol. 77, no. 50, 1977, XP002138662

## Description

### TECHNICAL FIELD

This invention generally relates to compositions comprising redox-active therapeutics, particularly tocotrienol quinones, for preventing, or treating cerebral ischemia in a subject experiencing a cerebral ischemic event, experiencing symptoms associated with or due to a cerebral ischemic condition, or being at risk for a cerebral ischemic condition. The invention also relates to compositions comprising tocotrienol quinones for protecting brain tissue from cerebral ischemia after trauma to the head or brain in subjects in need of such treatment. The invention also relates to the prevention or treatment of cerebral ischemia with an effective amount of a redox-active therapeutic, such as a tocotrienol quinone, to minimize the size and severity of the infarct in the brain of the subject. This invention relates to the treatment and prevention of stroke; this invention does not relate to the treatment, amelioration or preventionof a mitochondrial disease such as "mitochondrial myopathy, encephalopathy, lactic acidosis, and stroke" (MELAS), where stroke is one of the symptoms of the mitochondrial disease.

### BACKGROUND

Ischemia may be defined as the loss of blood flow to a tissue. Cerebral ischemia, also known as stroke, is the interruption or reduction of blood flow in the arteries feeding the brain. Loss of blood flow to a particular vascular region is known as focal ischemia; loss of blood flow to the entire brain is known as global ischemia. When deprived of blood, and thus, oxygen and glucose, brain tissue may undergo ischemic necrosis or infarction. The metabolic events thought to underlie such cell degeneration and death include: energy failure through ATP depletion; cellular acidosis; glutamate release; calcium ion influx; stimulation of membrane phospholipid degradation and subsequent free-fatty-acid accumulation; and free radical generation.

Tocopherols and tocotrienols, while generally similar in overall chemical structure, may vary in biological function. Alpha-tocopherol is generally considered the most biologically active form of vitamin E; it is also the most abundant in adult human serum (Neuzil et al., (1998) Care. Drugs. Ther. 12:421-423; Strohschein et al., (1998) Anal. Chem. 70:13-18; Gonzalez (1990) Med. Hypothes. 32:107-110). Alpha-tocopherol has a greater antioxidant activity than the other tocopherols (Fukuzawa et al., (1982) Lipids 17:511-13). Alpha-tocopherol, but not beta-tocopherol, inhibits protein kinase C function (Ricciarelli et al., (1998) Biochem. J. 334:243-249). However, both alpha- and beta-tocopherol inhibit porcine pancreatic phospholipase A2 activity (Grau et al., (1998) Chem. Phys. Lipids 91:109-118). Alpha-, beta-, gamma- and delta-tocopherol were all able to inhibit superoxide generation by neutrophils (Kanno et al., (1996) Free Radic. Res. 24:181-189). Delta-tocopherol has only one hundredth of the activity of natural alpha-tocopherol in the Evans resorption sterility test for vitamin E.

Khanna et al. describe neuroprotective properties of the natural vitamin E alpha-tocotrienol (Stroke (2005); 36, e144-e152). Stroke-dependent brain tissue damage was studied in 12-Lox-deficient mice and spontaneously hypertensive rats orally supplemented with alpha-tocotrienol. In neuronal cells, nM/L concentrations of alpha-tocotrienol, but not alpha-tocopherol, blocked glutamate-induced death by suppressing early activation of c-Src kinase and 12-lipoxygenase.

International application WO 00/078296 discloses a method for the treatment or prophylaxis of glutamate and/or induced neurodegenerative disorders and neuronal damage which are initiated by ischemia, reperfusion, trauma or massive bleeding.

International application WO 99/025336 discloses the prevention of restenosis using an angioplastic procedure comprising the coating of the exterior surface of an angioplastic balloon with a composition comprising a tocotrienol and performing the arterial angioplastic procedure, such that a prophylactically effective amount of the composition is transferred to the interior surface of the artery.

In the treatment of cerebral ischemia, free radical scavengers/antioxidants have been used to improve cerebral blood flow and/or neurological outcome. In general, the effects of these compounds on infarct volume have been inconsistent; see U.S. Pat. No. 5,872,108. For example, superoxide dismutase inhibitors have been found to reduce infarct volume only when injected intracerebroventricularly (Kinouchi et al., (1991) Proc. Natl. Acad. Sci. USA 88:11158-11162). Other compounds, such as lubeluzole, have been shown to have clinical benefit for cerebral ischemia but with a very narrow margin of safety (Diener et al., (1996) Stroke 27:76-81).

Cerebral ischemia is one of the major causes of human neurological morbidity and mortality with poor prognosis associated with stroke recovery. Thus, a need remains for identification of effective compositions which aid in the survival and recovery of cells during injury associated with cerebral ischemia or for mammalian subjects at risk for injury associated with cerebral ischemia.

### DISCLOSURE OF THE INVENTION

The present invention relates to compositions for the treatment and prevention of cerebral ischemia in a mammalian subject. This invention relates to the treatment and prevention of stroke and not to the treatment, amelioration, or prevention of a mitochondrial disease where stroke is one of the symptoms of the mitochondrial disease.

The present invention provides a composition for use in treating, preventing, and/or ameliorating neuronal damage associated with a cerebral ischemic event or hypoxia in a mammalian subject in need of such treatment, said composition comprising an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof; with the proviso that said subject does not suffer from a mitochondrial disease.

The present invention also provides a composition for use in treating and/or ameliorating a cerebral ischemic event or hypoxia in a mammalian subject in need of such treatment, said composition comprising an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof; with the proviso that said subject does not suffer from a mitochondrial disease where stroke is one of the symptoms of the mitochondrial disease.

In another embodiment, the present invention provides compositions for use in treating and/or ameliorating a cerebral ischemic or hypoxic condition, in a mammalian subject in need of such treatment, comprising administering to the subject an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof, with the proviso that the subject does not suffer from or have a mitochondrial disease.

In another embodiment, the present invention provides compositions for use in treating and/or ameliorating a cerebral ischemic or hypoxic condition, in a mammalian subject in need of such treatment, comprising administering to the subject an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof, with the proviso that the subject has not been administered an effective amount of one or more compounds of Formula I within about one week prior to a cerebral ischemic event or hypoxia, within about one month prior to a cerebral ischemic event or hypoxia, within about three months prior to a cerebral ischemic event or hypoxia, within about six months prior to a cerebral ischemic event or hypoxia, or within about one year prior to a cerebral ischemic event or hypoxia. In another embodiment, the present invention provides compositions for use in treating and/or ameliorating a cerebral ischemic or hypoxic condition, in a mammalian subject in need of such treatment, comprising administering to the subject an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof, with the proviso that the subject has never been administered an effective amount of one or more compounds of Formula 1. As used herein, a compound of Formula I is denoted as: 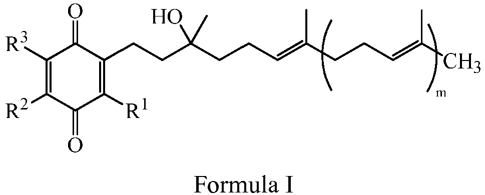 where,
R¹, R², and R³ are independently of each other hydrogen, (C₁-C₆)alkyl, or (C₁-C₆)alkoxy; and m is an integer between 1 and 12, inclusive;
or any stereoisomer, mixture of stereoisomers, prodrug, metabolite, salt, crystalline form, non-crystalline form, hydrate or solvate therof.

The present invention provides compositions for use in preventing a cerebral ischemic or hypoxic condition, in a mammalian subject in need of such prevention, comprising administering to the subject an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof; with the proviso that said subject does not suffer from a mitochondrial disease where stroke is one of the symptoms of the mitochondrial disease.

In another embodiment, the present invention provides compositions for use in preventing a cerebral ischemic or hypoxic condition, in a mammalian subject in need of such treatment, comprising administering to the subject an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof, with the proviso that the subject does not suffer from or have a mitochondrial disease.

In another embodiment, the present invention provides compositions for use in preventing a cerebral ischemic or hypoxic condition, in a mammalian subject in need of such prevention comprising administering to the subject an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof, with the proviso that the subject has not been administered an effective amount of one or more compounds of Formula I within about one week prior to a cerebral ischemic event or hypoxia, within about one month prior to a cerebral ischemic event or hypoxia, within about three months prior to a cerebral ischemic event or hypoxia, within about six months prior to a cerebral ischemic event or hypoxia, or within about one year prior to a cerebral ischemic event or hypoxia. In another embodiment, the present invention provides compositions for use in preventing a cerebral ischemic or hypoxic condition, in a mammalian subject in need of such prevention, comprising administering to the subject an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof, with the proviso that the subject has never been administered an effective amount of one or more compounds of Formula I.

The present invention provides compositions for use in treating and/or ameliorating the symptoms of a cerebral ischemic event or hypoxia in a mammalian subject, comprising administering to the subject an effective amount of a tocotrienol quinone or single steroisomer or mixtures of stereoisomers thereof, of the following structure:

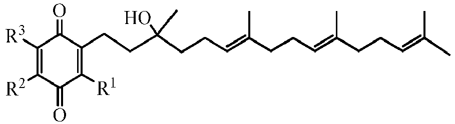

| | | | |
|---|---|---|---|
| Alpha-Tocotrienol quinone | R¹= CH₃ | R²= CH₃ | R³= CH₃ |
| Beta-Tocotrienol quinone | R¹= CH₃ | R²= H | R³= CH₃ |
| Gamma-Tocotrienol quinone | R¹= H | R²= CH₃ | R³= CH₃ |
| Delta-Tocotrienol quinone | R¹= H | R²= H | R³= CH₃ |

and by said administering, reducing neuronal damage related to said cerebral ischemic or hypoxic condition.

In other embodiments, the tocotrienol quinone is alpha-tocotrienol quinone. In other embodiments, the tocotrienol quinone is beta-tocotrienol quinone. In other embodiments, the tocotrienol quinone is gamma-tocotrienol quinone. In other embodiments, tocotrienol quinone is delta-tocotrienol quinone.

In other embodiments, the subject is administered an effective amount of a mixture of two or more tocotrienol quinones selected from alpha-tocotrienol quinone, beta-tocotrienol quinone, gamma-tocotrienol quinone and delta-tocotrienol quinone.

In some embodiments, the present invention provides compositions for use in treating and/or ameliorating the symptoms of a cerebral ischemic or hypoxic condition in a mammalian subject, comprising administering to the subject an effective amount of a composition comprising one or more compounds selected from the group consisting of alpha-tocotrienol quinone, beta-tocotrienol quinone, gamma-tocotrienol quinone and delta-tocotrienol quinone. In some particular embodiments, the present invention provides compositions for use in treating and/or ameliorating the symptoms of a cerebral ischemic or hypoxic condition in a mammalian subject, comprising administering to the subject an effective amount of a composition comprising alpha-tocotrienol quinone.

In other embodiments, the present invention provides compositions for use in preventing a cerebral ischemic or hypoxic condition in a mammalian subject, comprising administering to the subject an effective amount of a composition comprising one or more compounds selected from the group consisting of alpha-tocotrienol quinone, beta-tocotrienol quinone, gamma-tocotrienol quinone and delta-tocotrienol quinone with the proviso that the subject is not suffering from a mitochondrial disease where stroke is one of the symptoms of the mitochondrial disease, or with the proviso that the subject is not suffering from a mitochondrial disease, or with the proviso that the subject has not been administered an effective amount of one or more compounds selected from the group consisting of alpha-tocotrienol quinone, beta-tocotrienol quinone, gamma-tocotrienol quinone and delta-tocotrienol quinone within about the past week, month, three months, six months, or year, or with the proviso that the subject has never been administered an effective amount of one or more compounds selected from the group consisting of alpha-tocotrienol quinone, beta-tocotrienol quinone, gamma-tocotrienol quinone and delta-tocotrienol quinone. In some particular embodiments, the present invention provides compositions for use in preventing a cerebral ischemic or hypoxic condition in a mammalian subject, comprising administering to the subject an effective amount of a composition comprising alpha-tocotrienol quinone, with the proviso that the subject is not suffering from a mitochondrial disease where stroke is one of the symptoms of the mitochondrial disease, or with the proviso that the subject is not suffering from a mitochondrial disease, or with the proviso that the subject has not been administered an effective amount of one or more compounds selected from the group consisting of alpha-tocotrienol quinone, beta-tocotrienol quinone, gamma-tocotrienol quinone and delta-tocotrienol quinone within about the past week, month, three months, six months, or year, or with the proviso that the subject has never been administered an effective amount of one or more compounds selected from the group consisting of alpha-tocotrienol quinone, beta-tocotrienol quinone, gamma-tocotrienol quinone and delta-tocotrienol quinone.

In some embodiments of the present invention, the cerebral ischemic or hypoxic condition is secondary to an occlusion of the cerebral vasculature and in other embodiments the occlusion is due to a thromboembolism. In further embodiments, the cerebral ischemia or hypoxia is due to a spasm of the coronary vasculature, with the proviso that the subject is not suffering from a mitochondrial disease where stroke subsequent to spasm of the coronary vasculature is one of the symptoms of the mitochondrial disease, or with the proviso that the subject is not suffering from a mitochondrial disease, or with the proviso that the subject has not been administered an effective amount of one or more compounds of Formula I within about the past week, month, three months, six months, or year, or with the proviso that the subject has never been administered an effective amount of one or more compounds of Formula I. In additional embodiments, the cerebral ischemic or hypoxic condition is secondary to a cessation of cardiac function. In further embodiments, the cerebral ischemic or hypoxic condition is secondary to a cardiopulmonary bypass procedure. In yet additional embodiments, the cerebral ischemic or hypoxic condition is secondary to a hemorrhagic event in the cerebral vasculature.

In yet other aspects, a tocotrienol quinone composition for use in the methods described herein comprises at least about 50%, at least about 55%, at least about 60%, at least about 65%, at least about 70%, at least about 75%, at least about 80%, at least about 85%, at least about 90%, at least about 95%, or at least about 98% tocotrienol quinone. In other aspects, an alpha-tocotrienol quinone composition for use in the methods described herein comprises at least about 50%, at least about 55%, at least about 60%, at least about 65%, at least about 70%, at least about 75%, at least about 80%, at least about 85%, at least about 90%, at least about 95%, or at least about 98% alpha-tocotrienol quinone

In some embodiments of the present invention, a composition for use in the methods described herein comprises a tocotrienol quinone in a range of about 1 to about 1000 mg per kg body weight of said mammalian subject. In yet other embodiments, a composition for use in the methods described herein comprises a tocotrienol quinone in a range of about 1 to about 500 mg per kg body weight of said mammalian subject. In further embodiments, a composition comprises a tocotrienol quinone in a range of about 5 to about 100 mg per kg body weight of said mammalian subject.

In some embodiments of the present invention, a composition for use in the methods described herein comprises an alpha tocotrienol quinone in a range of about 1 to about 1000 mg per kg body weight of said mammalian subject. In yet other embodiments, a composition for use in the methods described herein comprises an alpha-tocotrienol quinone in a range of about 1 to about 500 mg per kg body weight of said mammalian subject. In further embodiments, a composition for use in the methods described herein comprises an alpha- tocotrienol quinone in a range of about 5 to about 100 mg per kg body weight of said mammalian subject.

In some embodiments, the composition is a pharmaceutical composition. In other embodiments, the composition is a medical food. In some embodiments, the administering of the composition is via an enteral route. In other embodiments, the administering of the composition is via an oral route. In yet further embodiments, the administering is via a parenteral route.

In other aspects, the present invention provides tocotrienol quinone compositions comprising a composition enriched with alpha-tocotrienol quinone in an amount effective to reduce neuronal damage related to a cerebral ischemic or hypoxic condition, when administered to a patient in need thereof. In some embodiments, the present invention provides tocotrienol quinone compositions comprising a composition enriched with alpha-tocotrienol quinone in an amount effective to reduce neuronal damage related to a cerebral ischemic or hypoxic condition, when administered to a patient in need thereof, with the proviso that the patient is not suffering from a mitochondrial disease where cerebral ischemia or hypoxia is one of the symptoms of the mitochondrial disease, or with the proviso that the subject is not suffering from a mitochondrial disease, or with the proviso that the subject has not been administered an effective amount of a tocotrienol quinone composition comprising a composition enriched with alpha-tocotrienol quinone within about the past week, month, three months, six months, or year, or with the proviso that the subject has never been administered an effective amount of a tocotrienol quinone composition comprising a composition enriched with alpha-tocotrienol quinone.

### BRIEF DESCRIPTION OF THE DRAWINGS

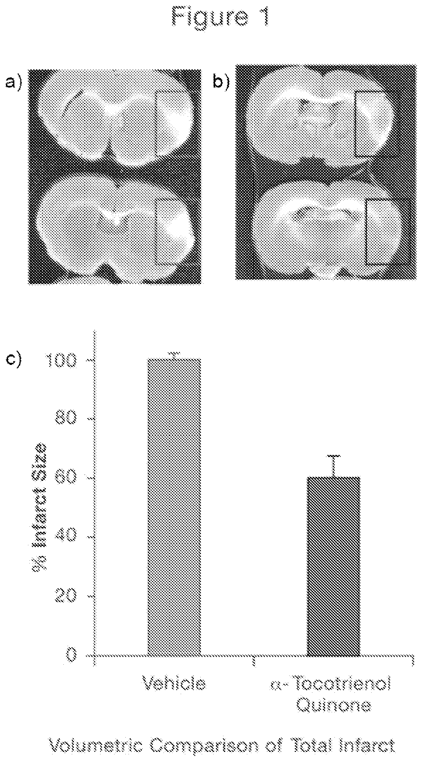

Figure 1 shows the effect of alpha-tocotrienol quinone on the volumetric comparison (c) of total infarct with administration of alpha-tocotrienol quinone versus vehicle at reperfusion as described in Example 2. The images of the brain slices of rats dosed with vehicle (a) or with alpha-tocotrienol quinone (b) at reperfusion are also provided; the boxes indicate the area of the infarct.

### MODES FOR CARRYING OUT THE INVENTION

The present invention generally relates to tocotrienol quinones that can be used in pharmaceutical compositions that are protective in cerebral ischemia (stroke) or hypoxia. The present invention provides compositions for use in preventing or treating cerebral ischemia or hypoxia, such as for example, by reducing neuronal cell death, reducing tissue edema, and/or reducing cognitive dysfunction associated with a cerebral ischemic or hypoxic disorder. The present invention provides compositions for use in reducing symptoms and/or conditions associated with a cerebral ischemic or hypoxic condition, such as, for example, infarct size, tissue edema or cognitive disorder associated with the presence of micro-emboli or a hypoxic condition. The present invention does not relate to the treatment of stroke in a subject suffering from a mitochondrial disease. In particular, the present invention does not relate to the treatment of stroke in a subject suffering from a mitochondrial disease, where stroke is one of the symptoms of the mitochondrial disease.

The present invention provides tocotrienol quinone compositions comprising at least about 50% alpha-tocotrienol quinone, at least about 55% alpha-tocotrienol quinone, at least about 60% alpha-tocotrienol quinone, at least about 65% alpha-tocotrienol quinone, at least about 70% alpha-tocotrienol quinone, at least about 75% alpha-tocotrienol quinone, at least about 80% alpha-tocotrienol quinone, at least about 85% alpha-tocotrienol quinone, at least about 90% alpha-tocotrienol quinone, at least about 95% alpha-tocotrienol quinone, or at least about 98% alpha-tocotrienol quinone. As used herein, an "active ingredient" is one that is able to treat or prevent cerebral ischemia or hypoxia in a mammalian subject. In some embodiments, tocotrienol quinone compositions comprise alpha-tocotrienol quinone as the sole active ingredient. In preferred embodiments, an active ingredient is able to reduce neuronal damage associated with cerebral ischemia or hypoxia at least about 20%, at least about 30%, at least about 40%, at least about 50%, at least about 60%, at least about 70%, at least about 80%, at least about 85%, and even more preferably at least about 90%, in experimental models such as those described herein. In some embodiments, the reduction of neuronal damage is measured by comparison of the volume of the treated experimental model system versus the untreated (control) experimental model system.

In additional preferred embodiments, the tocotrienol quinone composition comprises alpha-tocotrienol quinone in an amount effective to reduce neuronal cell death, reduce infarct size, reduce tissue edema associated with the cerebral ischemic or hypoxic condition, and/or reduce cognitive dysfunction, and may further comprise tocopherol quinone. In other preferred embodiments, the tocotrienol quinone compositions of the present invention comprise additional active ingredients. In some embodiments the compositions comprising the tocotrienol quinone and additional ingredient(s) provide a synergistic effect. Tocotrienol quinone and an additional ingredient are considered to be synergistic when their combined effect is greater than an additive effect of the individual effects. In other embodiments, the compositions comprise alpha-tocotrienol quinone and an additional ingredient providing a synergistic effect.

The subject compounds can be evaluated and validated as pharmacologically efficacious using in vitro assays based on a variety of different cell lines known in the art, as well in vivo via appropriate animal or whole organ assays, such as the isolated heart model of cardiac function, the rat air pouch model for inflammation, the rat middle-cerebral artery occlusion (MCAO) stroke model, and others known to those of skill in the art.

The monitoring of the effect of a treatment can be followed with magnetic resonance imaging ("MRI"). Illustrative MRI methods include cell-specific imaging, magnetization transfer imaging ("MTI"), gadolinium-enhanced MRI, proton magnetic resonance spectroscopy (MRS), and diffusion-weighted imaging (showing dead brain tissue), perfusion-weighted imaging (showing oxygen-starved but live brain tissue), and functional MR imaging (fMRI). For example, the ischemic penumbra may be identified by observing differences in the abnormal region defined by perfusion-weighted imaging and diffusion-weighted imaging as described in Duong, T. Q. and Fisher, M., (2004) Curr. Atheroscl. Rep. 6:267-273. This difference in the defined regions is known as the perfusion-diffusion mismatch. The perfusion-diffusion mismatch region is presumed to approximate the ischemic penumbra. Vasoconstriction and vasospasm are usually detected using digital subtraction angiography as known in the art.

Additionally, regional oxidative stress and glucose metabolism in brain lesions may be evaluated by imaging as typified by positron emission tomography (PET). Novel functional, double imaging of redox and energy states in different stages of stroke-like lesions using PET with [⁶²Cu]-diacetyl-bis(*N*4-methylthiosemicarbazone (⁶²Cu-ATSM) and [¹⁸F]-fluorodeoxyglucose (¹⁸FDG) as described in Ikawa M. et al., (2009) Mitochondrion 9, 144-148 can be performed *in vivo.* ⁶²Cu-ATSM-PET can be applied to evaluate oxidative stress and cerebral blood flow, whereas ¹⁸FDG-PET can be applied to diagnose glucose metabolism in the stroke lesions.

In an illustrative embodiment disclosed herein an alpha-tocotrienol quinone composition is shown to reduce total infarct at the time of MCAO and at the time of reperfusion, when administered by gavage.

### Definitions

The term "alkyl" refers to saturated aliphatic groups including straight-chain, branched-chain, cyclic groups, and combinations thereof, having the number of carbon atoms specified, or if no number is specified, having up to 12 carbon atoms. One subset of alkyl groups is (C₁-C₆)alkyl which includes, but is not limited to, groups such as methyl, ethyl, n-propyl, isopropyl, butyl, n-butyl, isobutyl, sec-butyl, t-butyl, pentyl, n-pentyl, hexyl, cyclopropyl, cyclobutyl, cyclopentyl, cyclohexyl, and any other alkyl group containing between one and six carbon atoms, where additional (C₁-C₆)alkyl groups can be attached via any valence on the alkyl group with the proviso that the total number of carbons is six or smaller than six. Another subset of alkyl groups is (C₁-C₄)alkyl which includes, but is not limited to, groups as methyl, ethyl, n-propyl, isopropyl, butyl, n-butyl, isobutyl, sec-butyl, t-butyl, cyclopropyl, and cyclobutyl.

The term "alkoxy" refers to the group -O-alkyl, wherein alkyl is as defined herein. One subset of alkoxy groups is (C₁-C₆)alkoxy which includes, but is not limited to, groups such as methoxy, ethoxy, n-propoxy, iso-propoxy, cyclo-propoxy, n-butoxy, tert-butoxy, sec-butoxy, cyclo-butoxy, n-pentoxy, cyclopentoxy, n-hexoxy, cyclohexoxy, and 1,2-dimethylbutoxy. Another subset of alkoxy groups is (C₁-C₄)alkoxy which includes, but is not limited to, groups such as methoxy, ethoxy, n-propoxy, iso-propoxy, cyclo-propoxy, n-butoxy, tert-butoxy, sec-butoxy, and cyclo-butoxy.

"Cerebral Ischemia" or "cerebral ischemic" or "a cerebral ischemic condition" refer to a medical event which is pathological in origin, or to a surgical intervention which is imposed on a subject, wherein circulation to a region of the brain is impeded or blocked, either temporarily, as in vasospasm or transient ischemic attack (TIA) or permanently (absent medical intervention), as in thrombotic or embolic occlusion. The affected region is deprived of oxygen and nutrients as a consequence of the ischemic event. This deprivation leads to the injury of an infarct in the region affected. Ischemia occurs in the brain during, for example, a thromboembolic stroke, hemorrhagic stroke, cerebral vasospasm, head trauma, cardiac arrest, severe blood loss due to injury or internal hemorrhage, and other similar conditions that disrupt normal blood flow. It may also occur after a head trauma, since the pressure caused by edema presses against and flattens the arteries and veins inside the brain, thereby reducing their ability to carry blood through the brain. Cerebral ischemia may also occur as a result of macro- or micro-emboli, such as may occur subsequent to cardiopulmonary bypass surgery.

"Infarct" or "infarction" relates to a region of a tissue or organ subjected to ischemia and suffering the physiological sequelae of ischemia. Infarction results from a sudden insufficiency of arterial or venous blood supply due to, for example, emboli, thrombi, vascular torsion or pressure that produces a macroscopic area of necrosis. Infarction also relates to a region injured as a result of exposure to a hemorrhage.

By "increasing cerebral blood flow" is meant the act of improving clinical outcome by inducing a statistically or physiologically significant increase in cerebral blood flow in a treated subject relative to an untreated subject as determined using techniques which are well known in the art, such as vascular imaging, for example.

By "reducing infarct size" is meant the act of improving clinical outcome by inducing a statistically or physiologically significant reduction in infarct size in a treated subject relative to an untreated subject as determined using techniques which are well known in the art, such as vascular imaging, for example.

"Agents" are defined herein as compounds, mixtures, or formulations of compounds which are capable of preventing or treating cerebral ischemia or hypoxia, such as by reducing neuronal damage or symptoms thereof, associated with a cerebral ischemic or hypoxic condition and/or cell damage due to cerebral ischemia or hypoxia. "Amelioration" means the prevention, reduction, palliation, or a counter-acting of the negative aspects of an ischemic or hypoxic condition or ischemic or hypoxic state. Amelioration does not require a complete recovery or complete prevention of the cerebral ischemic or hypoxic condition. A compound or agent may provide protective activity prior to, simultaneously with, and/or after the cerebral ischemic or hypoxic event has occurred.

A "pharmaceutical composition" is a composition comprising an agent that is suitable for administration to a patient or subject. The pharmaceutical composition can also contain additional components, such as pharmaceutically acceptable excipients, pharmaceutically acceptable carriers, and pharmaceutically acceptable vehicles. A "prescription pharmaceutical composition" is a composition that requires a physician's prescription for administration. A pharmaceutical composition can comprise a compound of Formula I, such as a tocotrienol quinone, such as alpha-tocotrienol quinone, to be administered in a range of about 1 to about 1000 mg per kg body weight of said mammalian subject. A "nutritional composition" is a composition that comprises naturally occurring components, preferably found in the food supply, such as supplements, functional foods or food ingredients, admixed together with an agent such as a compound of Formula I, such as a tocotrienol quinone, such as alpha-tocotrienol quinone.

As used herein, an agent is said to be "cytoprotective" or to have "cytoprotective property" or "cytoprotective activity" if administration of the agent reduces and/or ameliorates symptoms of a cerebral ischemic or hypoxic condition and/or injury suffered by cells, tissues, organs and/or organisms that is induced secondary to cerebral ischemia or hypoxia. Cytoprotective activity and injury can be quantified in assays which measure results of injury such as death and inhibition of metabolic activity; these can be measured, for example, using appropriate fluorescent dyes, measuring enzyme activity and/or measuring intact cellular membrane in affected tissues by staining with appropriate indicators. Cytoprotective agents include cytoprotective tocotrienol quinones, metabolites thereof and derivatives thereof.

A "synergist" is defined as an agent or compound which when present results in a greater-than-additive increase, augmentation or enhancement of the effect of an agent or compound. In some cases, it may be difficult to determine which compound in a mixture is of primary importance and which only secondary. Thus, in a synergistic mixture of compounds, any of the active compounds within the mixture can be considered a synergist. A composition comprising "synergistic activity" or a "synergistic mixture" is a combination of compounds wherein the combined effect is greater than additive of the individual effects. Synergism may be apparent only at some ranges or concentrations.

By "effective amount" or "amounts effective to reduce neuronal damage associated with a cerebral ischemic or hypoxic conditions and/or symptoms due to cerebral ischemia or hypoxia" is meant that the cytoprotective agent or agents (e.g., tocotrienol quinones) is administered to the patient in an amount sufficient for reducing injury associated with a cerebral ischemic or hypoxic condition and/or symptoms due to cerebral ischemia or hypoxia, and/or in an amount sufficient to provide a final concentration in the affected tissues sufficient for reducing injury associated with a cerebral ischemic or hypoxic condition and/or symptoms due to cerebral ischemia or hypoxia. This amount includes, but is not limited to, a concentration which acts as a complete prophylaxis or treatment for a symptom of neuronal damage, or which acts as a partial prophylaxis or treatment for a symptom of neuronal damage. An "effective amount" is an amount sufficient to effect beneficial or desired results. An effective amount can be administered in one or more administrations. For purposes of this invention, an effective amount of a cytoprotective composition is an amount that is sufficient to ameliorate, stabilize, reverse, slow or delay the progression of injury in mammalian subjects i) at risk for a cerebral ischemic or hypoxic condition, or ii) associated with symptoms of a cerebral ischemic or hypoxic condition. Preferably, amelioration of injury due to a cerebral ischemic or hypoxic condition can be quantified by an assay measuring, for example, reduction in cell death and/or enzyme inactivity; and/or reduction of tissue edema; and/or reduction in cognitive disorder; and/or reduction of infarct size. In the case of injuries associated with a cerebral ischemic or hypoxic condition, the size and/or severity of an infarct in the brain of the subject may be determined, for example, by various noninvasive radiological procedures and/or by various symptomatic and diagnostic procedures known to those of skill in the art, such as magnetic resonance imaging (MRI), computerized tomography (CT) scan, positron emission tomography (PET), diffusion weighted imaging (DWI) and the like. Injuries associated with a cerebral ischemic or hypoxic condition also include cerebral edema and injuries that are associated with such edema. When used in relation to a cerebral ischemic or hypoxic condition, the term "effective" when describing a dose size, frequency, or duration, or the concept of dose "effectiveness," relates to a dosing which results in a reduction in the size and severity of an actual cerebral infarct, or to a probability that any such cerebral infarct, were it to occur, would be of reduced size and severity. Amelioration is preferably at least about 20%, preferably at least about 30%, preferably at least about 50%, more preferably at least about 70%, even more preferably at least about 80%, and even more preferably at least about 90% reduction in neuronal damage.

"Hypoxia," which is defined broadly as a condition under which a particular cell, organ or tissue receives an insufficient oxygen supply to allow normal function. More specifically, hypoxia can be measured as an average or mean environmental oxygen saturation level of less than 90% or less than about 90%. Hypoxia is a direct result of ischemia, since whenever blood supply is cut off, oxygen supply is also cut off. However, hypoxia can occur in other conditions, even if blood flow remains unaltered, including, but not limited to, carbon monoxide poisoning, drowning, suffocation and other forms of asphyxia.

The term "energetically-competent" refers to cells, cell lines or organisms which undergo aerobic respiration (oxidative metabolism). "Energetic incompetence" refers to the quality of cells incapable of undergoing aerobic respiration; such cells only perform anaerobic respiration (fermentation).

By "treatment" or "treating" is meant any treatment of a disease or disorder, in a mammal inhibiting the disease, that is, alleviating, arresting or suppressing the development of clinical symptoms; and/or relieving the disease, that is, causing the regression of clinical symptoms.

By "prevention" or "prophylaxis" is meant a regimen that prevents or protects against the disease or disorder, that is, causing, the clinical symptoms of the disease not to develop. Prevention may be partial or complete.

By "tocotrienol quinone composition" is meant a composition comprising alpha-tocotrienol quinone, beta-tocotrienol quinone, gamma-tocotrienol quinone, delta-tocotrienol quinone and/or mixtures thereof. The term tocotrienol quinone also includes single stereoisomers and mixtures of stereoisomers, salts, crystalline forms, non-crystalline forms, hydrates or solvates therof.

### General Methods

In certain embodiments the formulations of the present invention comprise tocotrienol quinones which can be produced synthetically from the respective tocotrienols by oxidation with suitable oxidizing agents, as for example ceric ammonium nitrate (CAN). Particularly, the formulations of the present invention comprise alpha-tocotrienol quinone (CAS Reg. No. 1401-66-7) produced by oxidation of essentially pure alpha-tocotrienol. A preferred process for the production of essentially pure alpha-tocotrienol or alpha-tocotrienol quinone has been described in co-owned US Patent Application Publication No. 2010/0105930 titled "Process for the Production of Alpha Tocotrienol and Derivatives".

Illustrative examples provided herein relate to compositions comprising alpha-tocotrienol quinone as the active ingredient for use as a cytoprotective agent against damage, injury and/or symptoms associated with cerebral ischemia or hypoxia.

The alpha-tocotrienol quinone compositions comprise alpha-tocotrienol quinone in amounts effective to ameliorate the injury and/or symptoms associated with cerebral ischemia or hypoxia. Alpha-tocotrienol quinone compositions may be enriched compositions comprising at least about 50% alpha-tocotrienol quinone, at least about 55% alpha-tocotrienol quinone, at least about 60% alpha-tocotrienol quinone, at least about 65% alpha-tocotrienol quinone, at least about 70% alpha-tocotrienol quinone, at least about 75% alpha-tocotrienol quinone, at least about 80% alpha-tocotrienol quinone, at least about 85% alpha-tocotrienol quinone, at least about 90% alpha-tocotrienol quinone and at least about 95% alpha-tocotrienol quinone.

In illustrative examples disclosed herein, alpha-tocotrienol quinone composition was able to reduce total infarct size by 40 % when administered by gavage at 500mg/kg at the time of MCAO and when administered at reperfusion (see Example 2 and FIG. 1, respectively).

In the present invention, a nutritional composition will comprise a compound such as a tocotrienol quinone, such as alpha-tocotrienol quinone, to be administered in a range of about 1 to about 50 mg per kg body weight of said mammalian subject. In additional embodiments, a nutritional composition will comprise a compound such as a tocotrienol quinone, such as alpha-tocotrienol quinone, to be administered at a lower limit of at least about 1, about 1.5, about 2, about 2.5, about 5, about 7.5, about 10, about 12.5, about 15, about 17.5, about 20, about 22.25, about 25, about 30, about 35, about 40, about 45 or about 50 mg per kg body weight of said mammalian subject. A pharmaceutical composition will comprise a compound such as a tocotrienol quinone, such as alpha-tocotrienol quinone, to be administered in a range of about 1 to about 1000 mg per kg body weight of said mammalian subject. In additional embodiments, a pharmaceutical composition will comprise a compound such as a tocotrienol quinone, such as alpha-tocotrienol quinone, to be administered at a lower limit of at least about 1, about 10, about 20, about 30, about 40, about 50, about 60, about 70, about 80, about 90, about 100, about 200, about 300, about 400, or about 500 mg per kg body weight of said mammalian subject. In additional embodiments, a pharmaceutical composition will comprise a compound such as a tocotrienol quinone, such as alpha-tocotrienol quinone, to be administered in a range of about 5 to about 500 mg per kg body weight of said mammalian subject.

### Methods of Using Compounds of the Invention

The compositions of the present invention are administered to a subject in amounts to reduce neuronal cell damage. The subject may be experiencing a cerebral ischemic or hypoxic condition, may be experiencing symptoms associated with or due to a cerebral ischemic or hypoxic condition or may be at risk for a cerebral ischemic or hypoxic condition. The subject is not being treated for the prevention of an ischemic or hypoxic condition resulting from a mitochondrial disease such as MELAS.

In one aspect, compositions of the present invention are used to prevent neuronal damage in a mammalian subject at risk of developing injury due to a cerebral ischemic or hypoxic condition, e.g. for example, by an infarction in the brain. The reduction of neuronal damage relates to minimizing the extent and/or severity of injury in the brain associated with or due to a cerebral ischemic or hypoxic condition by ameliorating or reducing the injury that would otherwise occur. An alpha-tocotrienol quinone composition is administered in some embodiments to a subject. The amount administered and the duration of the treatment are effective to minimize the size and/or severity of the neuronal damage in the mammalian subject as measured by for example, reduction in neuronal cell death and/or reduction in tissue edema associated with a cerebral ischemic or hypoxic condition and/or reduction in cognitive disorder and/or reduction in infarct size. Thus, it is anticipated that as a result of such treatment the size and/or severity of any neuronal damage that develops is minimized.

The composition is used in prophylactic treatments for neuronal damage including cell death and/or presence of tissue edema and/or cognitive dysfunction and/or cerebral infarcts which may be due to ischemic, hypoxic/anoxic, or hemorrhagic events. Compositions of the present invention are administered to a subject at risk of experiencing neuronal damage associated or due to a cerebral ischemic or hypoxic condition, and prevent it or ameliorate the severity of the damage, should it occur. The composition is intended for a subject at risk of neuronal damage that is associated with, or results from, an acute or chronic medical condition. Such conditions might arise as a result of medical or surgical treatment planned for the subject (e.g., angioplasty) or as a result of an emergent medical condition such as a stroke or severe blood loss. Other conditions which place a subject at risk for neuronal damage associated with a cerebral ischemic condition include a genetic predisposition to stroke or a condition that is understood to increase the probability of incurring a cerebral infarct such as atherosclerosis, previous stroke or transient ischemic or hypoxic attacks, diabetes mellitus, hypertension, hypercholesterolemia, a history of smoking and may also include schizophrenia, epilepsy, and neurodegenerative disorders. Diagnostic and/or pathological characterization of stroke victims has identified numerous additional medical conditions producing stroke that are widely known to practitioners of internal and neurological medicine.

Additional medical conditions that place a subject at risk for neuronal damage associated with or due to a cerebral ischemic or hypoxic condition include, but are not limited to, vasculitis (including collagen vascular disease, temporal (giant cell) arteritis, polyarteritis nodosa, Wegener's granulomatosis, Takayasu's arteritis, and vasculitis associated with syphilis); meningitis (including, but not limited to, meningitis caused by tuberculosis, fungi, syphilis, bacteria, or herpes, including herpes zoster); arterial dissection (e.g., carotid, vertebral, or intracranial arteries at the base of the brain); hematologic disorders (including but not limited to, polycythemia, thrombocytosis, thrombosis, thrombocytopenic purpura, disseminated intravascular coagulation, dysproteinemias, and hemoglobinopathies (such as sickle cell disease)); vascular damage induced by cocaine or amphetamines; moyamoya disease; fibromuscular dysplasia; Binswanger's disease; embolism (including cardiac sources, e.g., dysrhythmia, coronary heart disease, rheumatic heart disease, etc.); coronary artery bypass graft (CABG), and atherothrombotic arterial sources (including but not limited to bifurcation of common carotid artery, carotid siphon, distal vertebral artery, and aortic arch).

Other medical conditions that place a subject at risk for neuronal damage associated with or due to a cerebral ischemic or hypoxic condition include, but are not limited to conditions associated with a hypercoagulable state secondary to systemic disease; carcinoma (especially pancreatic); eclampsia; oral contraceptives; lupus; thrombotic diseases such as factor C or S deficiency and Factor V mutation such as Factor V Leiden; vasoconstriction, including vasospasm (e.g., cerebral vasospasm following subarachnoid hemorrhage) and reversible cerebral vasoconstriction (e.g., idiopathic, migraine, eclampsia, trauma); and venous conditions (including dehydration, pancranial infection, postpartum and postoperative states and systemic cancer).

Medical conditions that place a subject at risk of neuronal damage associated with or due to a cerebral ischemic or hypoxic condition due to intracranial hemorrhage include, but are not limited to, spontaneous intracerebral hemorrhage (e.g., hypertensive, amyloid angiopathy); ruptured aneurysm (e.g., saccular, mycotic); ruptured arteriovenous malformation; drug use (e.g., cocaine, amphetamines); trauma; bleeding with brain tumors; systemic bleeding disorders (including anticoagulation therapy) and hemorrhagic infarction.

The present invention provides compositions for human and nonhuman mammal subjects. A nonhuman mammal is identified as a subject if the animal is to be subjected to procedures that induce cerebral ischemic or hypoxic condition and lead to, for example, a cerebral infarction, or is to undergo surgical or invasive procedures comparable to those identified in the preceding paragraph for human subjects.

In the present invention, the tocotrienol quinone composition is administered to a mammalian subject. In the case where an individual has been diagnosed as having suffered an ischemic or hypoxic event, such as a stroke or a result of suffocation, the administration of a composition of the present invention should begin as soon as possible after the ischemic or hypoxic event occurred, preferably within a few days (e.g., within about 1, about 2 or about 3 days), or more preferably within a few hours (e.g., less than about 12 hours, less than about 8 hours, less than about 6 hours, less than about 4 hours, less than about 2 hours, or less than about 1 hour) of the event. In the case where an individual is at risk of developing an infarct as a result of impending surgical or similar intervention or is otherwise at high risk of infarction, administration of the compositions of the present invention preferably begins as soon as the decision planning the intervention is made or the risk identified. In either case, the duration of the treatment is a few days, several days, or a few weeks, determined by the time frame in which ischemic or hypoxic injury is understood, or expected, to occur.

The size of the dose of the tocotrienol quinone composition to be administered depends on the route of administration and the medical condition of the subject, as well as other individual parameters of the subject such as age, size and weight. Administration of the composition to an individual having suffered an ischemic or hypoxic event represents the most acute situation of those considered in this invention, since the ischemic or hypoxic event has already occurred and the need for immediate minimization of injury from infarction is extreme. This may necessitate a particular size of dose appropriate for the circumstances. A human subject or nonhuman animal scheduled for imminent surgery or medical intervention is in a somewhat less acute medical state. As a result the size of each dose may be different, and may be open to somewhat greater variation and still be within the practice of the invention. In general, the sizes of each dose for administration are described herein. Dose sizes for nonhuman mammals generally fall in the same range on a mg/kg basis.

For the reasons just summarized, the frequency of dosing may also vary. A more acute medical status may suggest a different dosing regime than one that is somewhat less acute. In general, the invention may be practiced by administering doses at a frequency ranging from about once or twice per week to about once, twice, three times, or four times daily, or by administering doses via a continuous infusion.

Likewise for the reasons given above, the duration of dosing is subject to variability. For example, a stroke victim, being in an extremely acute medical condition, requires the effect of the method of the invention to be realized as early as possible. This clearly dictates a course of dosing that emphasizes a short duration. A subject facing scheduled surgery or medical intervention, or being otherwise at high risk for a stroke, having a less acute medical status, may be subjected to dosing for a different, generally longer, duration. In general, a victim of stroke may be administered the composition for a duration ranging from about 3 days to about 10 days. On the other hand, a subject who is about to undergo medical or surgical treatment which enhances the risk of a cerebral ischemic or hypoxic condition in the brain may undergo dosing for a duration ranging from about 5 days to about 14 days prior to the intervention. A subject who is at risk for developing a cerebral infarct associated with a chronic medical condition such as a genetic predisposition to stroke, diabetes mellitus, hypertension, hypercholesterolemia, and a history of smoking may be treated preventatively for a longer duration.

The compositions of the invention are administered in an effective amount. With regard to a cerebral ischemic or hypoxic condition, an effective amount is one sufficient to reduce neuronal damage resulting from the cerebral ischemic or hypoxic condition. A reduction of neuronal damage is any prevention of injury to the brain which otherwise would have occurred in a subject experiencing a cerebral ischemic event or hypoxia absent the treatment of the invention. Several physiological parameters may be used to assess reduction of brain injury, including, but not limited to, a smaller infarct size, improved cerebral regional blood flow and decreased intracranial pressure, for example, as compared to pretreatment patient parameters, untreated cerebral ischemic or hypoxic patients or cerebral ischemic or hypoxic patients receiving a control alone. The size of an infarct in a human patient having suffered a stroke may be determined, for example, by various noninvasive radiological procedures known to those of skill in the field of medicine, especially to those of skill in radiology and neurology. Examples of methods available in the field include, but are not limited to, computerized tomography (CT) scanning, magnetic resonance imaging (MRI), positron emission tomography (PET), diffusion weighted imaging (DWI), ultrasonic imaging, and targeted radiotracer imaging.

The severity of an infarct in a human patient having suffered a stroke may be determined, for example, by various symptomatic and diagnostic procedures known to those of skill in the fields of medicine, especially to those of skill in neurology, hematology, and physical medicine, in addition to assessing the results of radiological and anatomical diagnosis that were discussed in the preceding paragraph. Kinetic, sensory, and cognitive behavior is affected in stroke patients. Medical diagnosis routinely includes such assessments in analysis of the status of stroke victims. For example, the effectiveness of the various doses of the claimed compositions may be assessed using standard measurements known in the art including, but not limited to, the Barthel Index, Modified Rankin Score, NIH Stroke Scale total, NIH Stroke Scale motor item, the number of days to discharge from the hospital, mortality and other neuropsychological battery scores.

In addition, stroke patients may be diagnosed by the methods of hematology. These may be used to assess the populations and cellular characteristics of immune cells in the circulation, as well as various enzymatic activities or cellular components from brain tissue. These activities or components are generally found in the blood of stroke victims but are typically absent or present at only low levels in subjects that have not suffered a stroke. Similar procedures may be applied to nonhuman mammals who have suffered ischemic or hypoxic injury as a result of medical or surgical procedures. The amounts or values of the various results obtained in these diagnostic tests may be evaluated with respect to values known in the various fields to represent normal or pathological states. As a result of evaluating the group of diagnostic results obtained as outlined above, the severity of the infarction may be assessed by workers of skill in the medical fields. The dose size, frequency, and the duration of treatment by the method of the present invention may be adjusted accordingly based on the severity of the infarction and the general medical condition of the patient.

The compositions, as described above, can be prepared as a medicinal preparation or in various other media, such as foods for humans or animals, including medical foods and dietary supplements. A "medical food" is a product that is intended for the specific dietary management of a disease or condition for which distinctive nutritional requirements exist. By way of example, but not limitation, medical foods may include vitamin and mineral formulations fed through a feeding tube to cancer or burn victims (referred to as enteral administration or gavage administration). A "dietary supplement" is a product that is intended to supplement the human diet and is typically provided in the form of a pill, capsule, tablet, or similar formulation. By way of example, but not limitation, a dietary supplement may include one or more of the following ingredients: vitamins, minerals, herbs, botanicals; amino acids, dietary substances intended to supplement the diet by increasing total dietary intake, and concentrates, metabolites, constituents, extracts or combinations of any of the foregoing. Dietary supplements may also be incorporated into food , including, but not limited to, food bars, beverages, powders, cereals, cooked foods, food additives and candies ; or other functional foods designed to promote cerebral health or to prevent cerebral ischemia or hypoxia. For animals, the compositions can be incorporated into the primary animal feed. If administered as a medicinal preparation, the composition can be administered, either as a prophylaxis or treatment, to a patient in any of a number of methods. The tocotrienol quinone compositions may be administered alone or in combination with other pharmaceutical agents and can be combined with a physiologically acceptable carrier thereof. In some embodiments, the tocotrienol quinone composition additionally comprises a pharmaceutically acceptable carrier. The effective amount and method of administration of the particular cytoprotective formulation can vary based on the individual subject, the stage of disease, and other factors evident to one skilled in the art. During the course of the treatment, the concentration of the subject compositions may be monitored to ensure that the desired level is maintained. The subject compositions may be compounded with other physiologically acceptable materials which can be ingested including, but not limited to, foods.

The compounds described herein can be formulated as pharmaceutical compositions by formulation with additives such as pharmaceutically acceptable excipients, pharmaceutically acceptable carriers, and pharmaceutically acceptable vehicles. Suitable pharmaceutically acceptable excipients, carriers and vehicles include processing agents and drug delivery modifiers and enhancers, such as, for example, calcium phosphate, magnesium stearate, talc, monosaccharides, disaccharides, starch, gelatin, cellulose, methyl cellulose, sodium carboxymethyl cellulose, dextrose, hydroxypropyl-beta-cyclodextrin, polyvinylpyrrolidinone, low melting waxes, ion exchange resins, and the like, as well as combinations of any two or more thereof. Other suitable pharmaceutically acceptable excipients are described in "Remington's Pharmaceutical Sciences," Mack Pub. Co., New Jersey (1991), and "Remington: The Science and Practice of Pharmacy," Lippincott Williams & Wilkins, Philadelphia, 20th edition (2003) and 21st edition (2005), incorporated herein by reference.

Pharmaceutical compositions of the invention may be in any form suitable for the intended method of administration, including, for example, a solution, a suspension, or an emulsion. Liquid carriers are typically used in preparing solutions, suspensions, and emulsions. Liquid carriers contemplated for use in the practice of the present invention include, for example, water, saline, pharmaceutically acceptable organic solvent(s), pharmaceutically acceptable oils or fats, and the like, as well as mixtures of two or more thereof. The liquid carrier may contain other suitable pharmaceutically acceptable additives such as solubilizers, emulsifiers, nutrients, buffers, preservatives, suspending agents, thickening agents, viscosity regulators, stabilizers, and the like. Suitable organic solvents include, for example, monohydric alcohols, such as ethanol, and polyhydric alcohols, such as glycols. Suitable oils include, for example, sesame oil, soybean oil, coconut oil, olive oil, safflower oil, cottonseed oil, and the like. For parenteral administration, the carrier can also be an oily ester such as ethyl oleate, isopropyl myristate, and the like. Compositions of the present invention may also be in the form of microparticles, microcapsules, liposomal encapsulates, and the like, as well as combinations of any two or more thereof.

Time-release or controlled release delivery systems may be used, such as a diffusion controlled matrix system or an erodible system, as described for example in: Lee, "Diffusion-Controlled Matrix Systems", pp. 155-198 and Ron and Langer, "Erodible Systems", pp. 199-224, in "Treatise on Controlled Drug Delivery", A. Kydonieus Ed., Marcel Dekker, Inc., New York 1992. The matrix may be, for example, a biodegradable material that can degrade spontaneously in situ and in vivo, for example, by hydrolysis or enzymatic cleavage, e.g., by proteases. The delivery system may be, for example, a naturally occurring or synthetic polymer or copolymer, for example in the form of a hydrogel. Exemplary polymers with cleavable linkages include polyesters, polyorthoesters, polyanhydrides, polysaccharides, poly(phosphoesters), polyamides, polyurethanes, poly(imidocarbonates) and poly(phosphazenes).

The compositions of the invention may be administered enterally, orally, parenterally, sublingually, by inhalation (e.g. as mists or sprays), rectally, or topically in dosage unit formulations containing conventional nontoxic pharmaceutically acceptable carriers, adjuvants, and vehicles as desired. For example, suitable modes of administration include oral, subcutaneous, transdermal, transmucosal, iontophoretic, intravenous, intraarterial, intramuscular, intraperitoneal, intranasal (e.g. via nasal mucosa), subdural, rectal, gastrointestinal, and the like, and directly to a specific or affected organ or tissue. For delivery to the central nervous system, spinal and epidural administration, or administration to cerebral ventricles, can be used. Topical administration may also involve the use of transdermal administration such as transdermal patches or iontophoresis devices. The term parenteral as used herein includes subcutaneous injections, intravenous, intramuscular, intrasternal injection, or infusion techniques. The compounds are mixed with pharmaceutically acceptable carriers, adjuvants, and vehicles appropriate for the desired route of administration. Oral administration is a preferred route of administration, and formulations suitable for oral administration are preferred formulations. The compositions described for use herein can be administered in solid form, in liquid form, in aerosol form, or in the form of tablets, pills, powder mixtures, capsules, granules, injectables, creams, solutions, suppositories, enemas, colonic irrigations, emulsions, dispersions, food premixes, and in other suitable forms. The compounds can also be administered in liposome formulations. Additional methods of administration are known in the art.

Injectable preparations, for example, sterile injectable aqueous or oleaginous suspensions, may be formulated according to the known art using suitable dispersing or wetting agents and suspending agents. The sterile injectable preparation may also be a sterile injectable solution or suspension in a nontoxic parenterally acceptable diluent or solvent, for example, as a solution in propylene glycol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols that are solid at room temperature but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

The compositions of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott, Ed., Methods in Cell Biology, Volume XIV, Academic Press, New York, N.W., p. 33 et seq (1976).

For administration, the formulations may conveniently be presented in unit dosage form and may be prepared by any methods well known in the art of pharmacy. Such methods include the step of bringing into association the active ingredients with the carrier which constitutes one or more accessory ingredients. In general, the formulations are prepared by uniformly and intimately bringing into association the active ingredients with liquid carriers or finely divided solid carriers or both, and then if necessary shaping the product. The invention also provides articles of manufacture and kits containing materials useful for treating or suppressing a cerebral ischemic or hypoxic condition. The article of manufacture comprises a container with a label. Suitable containers include, for example, bottles, vials, and test tubes. The containers may be formed from a variety of materials such as glass or plastic. The container holds a composition having an active agent which is effective for treating or suppressing a cerebral ischemic or hypoxic condition. The active agent in the composition is one or more of the compounds of the present invention. The label on the container indicates that the composition is used for treating or suppressing a cerebral ischemic or hypoxic condition, for example stroke, and may also indicate directions for either in vivo or in vitro use.

Kits may comprise any one or more of the compositions of the present invention. In some embodiments, the kit comprises the container described above. In other embodiments, the kit comprises the container described above and a second container comprising a buffer. It may further include other materials desirable from a commercial and user standpoint, including other buffers, diluents, filters, needles, syringes, and package inserts with instructions for performing any methods described herein.

In other aspects, the kits may be used for any of the methods described herein, including, for example, to treat an individual with a cerebral ischemic or hypoxic condition such as stroke, or to suppress a cerebral ischemic or hypoxic condition such as stroke in an individual.

The amount of the composition ingested, consumed or otherwise administered will depend on the desired final concentration. Typically, the amount of a single administration of the composition of the invention can be about 100 mg to about 500 mg per dose, or about 200 mg to about 1500 mg per day. Any of these doses can be further subdivided into separate administrations, and multiple dosages can be given to any individual patient.

The above-mentioned compositions and methods of administration are meant to describe but not limit the compositions of the present invention. The methods of producing various compositions and devices are within the ability of one skilled in the art and are not described in detail here.

Various assays, compositions and methods useful for identifying compositions and methods for reducing neuronal damage are provided in the Examples. The following examples are provided to illustrate, but not limit, the invention.

### EXAMPLES

### Example 1

### Cell-based assays for determining the ability of a composition to counteract ischemic-induced neuronal cell injury and cell death.

Insults to the brain that disrupt its blood supply, as in ischemia, or its oxygen supply, as in hypoxia (low oxygen) or anoxia (zero oxygen), rapidly cause neuronal imbalance leading to cell death (Flynn et al. (1989) Ischemia and Hypoxia, pp. 783-810, In: Basic Neurochemistry, Siegel et al. (Eds.), Raven Press, New York). Cerebral ischemic insults are modeled in animals by occluding vessels to, or within, the cranium (Molinari (1986) Experimental models of ischemic stroke, pp. 57-73, In: Stroke: Pathophysiology, Diagnosis and Management, Vol. 1, Barnett et al. (Eds.), Churchill Livingstone, N.Y.). In vitro models of ischemia use different means of oxygen and glucose deprivation, for example, by placing neuronal cultures into large anaerobic or hypoxic chambers and exchanging culture medium with oxygen-free and defined ionic composition media (Goldberg et al., (1990) Stroke 21:75-77). The toxic overstimulation of neuronal glutamate receptors, especially N-methyl-D-aspartate (NMDA) receptors, contributes to hypoxic-ischemic neuronal injury (Choi (1988) Neuron 1:623-634), ischemic induction of reactive oxygen species (ROS) (Watson et al., (1988) Ann. NY Acad. Sci. 59:269-281), excessive calcium influx (Grotta et al., (1988) Stroke 19:447-454), arachidonic acid increase (Siesjo (1981) J. Cereb. Blood Flow Metab. 1: 155-185), and DNA damage (MacManus et al., (1993) Neurosci. Lett. 164:89-92), causing a cascade of neurodegeneration.

Among various types of primary neuronal cultures, primary embryonic hippocampal neuronal culture is widely used for several reasons. The hippocampus is a source of a relatively homogenous population of neurons with well-characterized properties typical of central nervous system (CNS) neurons in general. Pyramidal neurons, the principal cell type in the hippocampus, have been estimated to account for 85% to 90% of the total neuronal population (Banker et al., (1998) Culturing Nerve Cells, 2nd edition, The MIT Press, Cambridge, Mass.). Also, the hippocampus exhibits a remarkable capacity for activity-dependent changes in synaptic function, such as long-term potentiation (Hawkins et al., (1993). Annu. Rev. Neurosci. 16:625-665).

Hippocampal cultures typically are prepared from 18- to 19-day fetal rats. At this age, the generation of pyramidal neurons, which begins in the rat at about E15, is generally complete. The tissue is easy to dissociate, the meninges are removed readily, and the number of glial cells still is relatively modest (Park et al., (2000) J Neurochem 74:114-124).

### Primary Cell Culture

The following protocol describes the procedure used to isolate and culture primary hippocampal neuronal cells from embryonic rat brain for use in the cell-based assays described herein.

Prior to cell isolation, long tip Pasteur pipettes with an opening of 1 mm, 0.4-0.5 mm, and 0.25 mm are fire-polished, cleaned with 70% ethanol, siliconized (Sigmacote, Sigma Chemical Cat. No. SL-2) and autoclaved. All other instruments for dissection are soaked in 70% ethanol at least 2 hr before the dissection. Also prior to cell isolation, culture flasks (T75 cm²) and plates are coated with poly-D-lysine (Sigma Chemical, Cat. No. P-6407). For the coating, 50 µg/ml poly-D-lysine is added to the flask or plate (5 mL per T75 cm² flask and 50 µL/well in a 96 well plate) for one hour. The flask or plate is then washed twice with sterile, distilled water and allowed to air dry in a culture hood for one hour before use. HBSS (Ca--Mg free) is prepared as follows: 10.0 mL 10xHBSS (Hank's CMF--Gibco #310-4180), 3.3 mL 0.3 M HEPES, pH 7.3, 10 mL of 0.35% sodium bicarbonate, 1.0 mL Penicillin/Streptomycin (100x) and 1.0 mL 100 mM pyruvate are mixed with 74.7 ml H₂O to make 100 mL of solution.

A pregnant rat (E 18-E 19) is euthanized with CO₂, and the uterus is removed. The embryos are removed from the sac, decapitated and their brains are removed. The brains are immersed in cold (4° C) BSS (Ca/Mg free) in a small petri dish. A dish (100-mm) is covered with paraffin to make a better surface for the dissection. The hippocampi are removed from the brains under a dissecting microscope and placed on the paraffin-covered dish. The meninges are stripped away and the dissected hippocampi are collected in a small petri dish in HBSS (Ca/Mg free).

The hippocampi from one litter are placed in a 15-mL centrifuge tube (generally 10-12 brains/litter), and the tube is filled with HBSS (Ca/Mg free). After centrifugation at 1000 rpm for 2 min using the desktop centrifuge, the supernatant is removed. 2 ml of HBSS (Ca/Mg free) is added to each tube and the tissue is triturated 2 times with a long tipped siliconized pipette with the three different opening sizes (total of 6-7 times). The trituration is started with a pipette with a normal opening size, and then smaller (half of size), then one with the smallest hole. After centrifugation at 1000 rpm for 2 minutes, the supernatant is discarded and 2 ml of Neurobasal/B27i (with antibiotics) is added to each tube. Neurobasal/B27i media contains Neurobasal medium (Life Technologies Cat No. 21103-049) with 1xB27 supplement (Life Technologies Cat No. 17504-044), 0.5 µM L-glutamine, 25 µM L-glutamic acid, and 1xPenicillin/Streptomycin. The cells are triturated 1 time with a long tip siliconized pipette with three different opening sizes. The trituration is started with a pipette with a normal opening size, then the smaller one (half of size) and finally the one with the smallest hole. Cell density is determined in a hemocytometer using the trypan blue exclusion method. A stock solution of 0.4% trypan blue in 0.9% NaCl is mixed one to one with a few drops of the cell suspension, and allowed to stand 4 minutes before counting the fraction of dye-excluding cells. A typical yield is 3x10⁵-6 x 10⁵ cells/brain.

The desired number of viable cells is added to poly-D-lysine-coated 12-well plates, flasks or MetTek culture dishes in Neurobasal/B27i, and incubated in air atmosphere with 5% CO₂ at 37°C. The cells are generally seeded at a density of 1.5x10⁶ cells per T75 cm² flask and at a density of ca.100,000 cells per well of a 12-well plate. Each T75 cm² flask received 15 mL of medium and each well of a 12-well plate received 1 mL of medium.

After three to four days in culture, half the media is removed from each well or flask, and an equal amount of fresh Neurobasal/B27m medium (Neurobasal medium with 1xB27 supplement, 0.5 µM L-glutamine), which contains 5 µM cytosine arabinoside (AraC), is added. Seven to eight days from the initial culture, half the media is removed from each well or flask, and an equal amount of fresh Neurobasal/B27m medium (no Ara-C) is added.

### Cell Injury Assay

In the following assay, ischemia is induced by anoxia-reoxygenation in cultured hippocampal neuronal cells and test agents are accessed for their potency and/or efficacy against ischemic-induced neuronal cell injury and cell death. The assay protocol is diagramed as follows:

Primary hippocampal neuronal cells are prepared and plated on poly-D-lysine coated 12-well plates as described above. The cells are cultured for 10-11 days as described above.

100 mL of LoG-Neurobasal medium in a T150 cm² flask is pre-equilibrated in the hypoxic chamber overnight and 20 mL of LoG-Neurobasal medium in a T75 cm² flask is pre-equilibrated in a standard incubator (5% CO₂) overnight. LoG-Neurobasal medium contains NoG-Neurobasal medium (no glucose) (Life Technologies, custom order) plus 0.5 mM glucose, 0.5 mM L-glutamine and 0.25 x Penicillin/ Streptomycin. 100 mL of Neurobasal/B27AO medium in a T150 cm² is pre-equilibrated in a standard incubator (5% CO₂) overnight. Neurobasal/B27A0 medium contains Neurobasal medium (Life Technologies, Cat. No. 21103-049) with 2xB27 minus AO supplement (Life Technologies, Cat. No. 10889-038), 0.5 mM L-glutamine, and 0.25xPenicillin/Streptomycin.

The equilibrated 100 ml of LoG-Neurobasal in T150 cm² flask is removed from the hypoxic chamber, and the medium is lightly bubbled with 100% N₂ for 30 min. to deoxygenate completely. Existing culture medium (Neurobasal/B27m) is aspirated from the cultured cells in each 12-well plate using the vacuum pump with an attached sterile glass Pasteur pipette. The cells are washed once with 2 mL of glucose free-BSS₀ (pH 7.4). Glucose free-BSS₀ (pH 7.4) contains 143.6 mM NaCl, 5.4 mM KCl, 1.8 mM CaCl₂, 0.8 mM MgSO₄, 1 mM NaH₂PO₄, 26.2 mM NaHCO₃, 10 mg/L phenol red and 0.25xPenicillin/Streptomycin.

The cultured neurons (10-11 days from initial culture) are replenished with deoxygenated LoG-Neurobasal (1 mL per well for each well of a 12-well plate). The test agents are added directly to each well (usually 3 concentrations of the compound plus positive control, each in triplicate). Generally, the test agents are dissolved in 100% DMSO. To reduce the DMSO effect on the cells, highly concentrated compounds are added in a small quantity (typically 200 x concentration). The concentration of DMSO in the culture does not exceed 0.5%.

The plates are placed, with their lids left ajar, in the anaerobic chamber for 5 hours. For normoxia controls, pre-equilibrated normoxic LoG-Neurobasal medium is added to each well and the plate replaced in the standard incubator (5% CO₂) for 5 hours. After 5 hours of hypoxia, the culture media is carefully aspirated and 2 mL of new oxygenated (pre-equilibrated) Neurobasal/B27AO is added to each well. Re-oxygenated medium is achieved by placing medium overnight in the culture incubator. The same test agents with the same concentrations are added back into the corresponding wells. The plates are placed in the cell culture incubator and re-oxygenated for 20-24 hours. After re-oxygenation for 20-24 hours, the number of live neurons is counted using the cell tracker green fluorescence method as follows. The culture medium is aspirated from each well of the 12-well plates and the neurons are washed once with 2 mL of HBSS (pre-warmed to 30-37°C); 25 mM HEPES, 100 mM NaCl, 5 mM KCl, 1.2 mM MgCl₂, 1.3 mM CaCl₂, 1.0 mM KH₂PO₄, pH 7.4, filter sterilized). 1 mL of 5 µM Cell Tracker Green fluorescent dye (Molecular Probes, Cat. No. 2925) dissolved in HBSS is added to the cells and the plates are placed in the dark at room temperature for 15 minutes. After washing the neurons once with 2 mL of HBSS, 1 mL of HBSS is added to each well, and fluorescent cells are counted using the fluorescent microscope.

### Example 2

### Animal Cerebral Infarct Assay

This assay was used to assess the efficacy of the test agents in protecting the brain against necrosis following cerebral ischemia induced in rats. Middle Cerebral Artery Occlusion (MCAO) is a widely used technique to induce transient focal cerebral ischemia in animal models. It has been demonstrated that the rat model of MCAO is an appropriate approximation of ischemic damage in humans. Furthermore, this model accurately represents the involvement of middle cerebral artery (MCA), the most affected vessel in human stroke, and also allows reperfusion as it happens in humans.

### Middle Cerebral Artery Occlusion (MCAO)

Male Sprague-Dawley rats (Hadan, Ind.) weighing 225-250 g were allowed free access to water and commercial rodent diet under standard laboratory conditions. The room temperature was maintained at 20-23 °C and room illumination was on a 12/12-hour light/dark cycle. The rats were acclimatized to the laboratory environment 5 to 7 days prior to the study.

Before surgery, the animals were not fasted and had free access to water. 4 hours prior to the surgery, animals were administered compounds of interest via oral gavage. Compounds were diluted in sesame oil for a final dose of 5ml/kg at 500mg/kg. Control animals received sesame oil; 4 hours post compound administration, the rats were anesthetized with 3.0% thiobutabarbital (Inactin; 100mg/kg) in 0.8% oxygen and placed in stereotaxic apparatus. The squamosal bone was removed to expose the middle cerebral artery (MCA). Sutures were placed in three neuroanatomically designated positions along the vessel. Once the sutures were in place, the timer was reset to "0". Reperfusion was initiated by withdrawal of sutures (the timer was again reset and this began the period termed "reperfusion"). The reperfusion lasted for an additional 5.5 hours at which time the animals were removed from the frame and the brain was removed by perfusing the animals with phosphate buffered saline (pH 7.4). The brain was placed in a brain matrix and coronal slices 1mm thick were cut and placed in TTC at room temperature for 10 minutes. The slices were transferred to a vial containing 10% formalin. The next day, the slices were scanned in a flatbed scanner and delivered to a data analyst who was blinded to the study, and infarct volumes of the digitized images were quantified using NIH Image J software.

### Drug Administration: Gavage Feeding

A standard rat gavage tube (Popper & Sons Inc, NY) was attached to a 3-cc hypodermic syringe. The animal was held by the shoulder in a vertical position. The feeding tube was placed into the mouth then advanced until it reached the stomach (the approximate insertion length of the tube was measured prior to the feeding). The content of the syringe was slowly delivered, and then the tube is withdrawn. The tocotrienol quinone was dissolved in sesame oil and delivered 4 hours prior to infarct.

Administration of alpha-tocotrienol quinone composition at the time of reperfusion resulted in 40% reduction of total infarct volume, total ischemic damage and cerebral edema relative to that of controls. Figure 1 shows images of the brain slices of rats dosed with vehicle (Figure 1(a)) or with alpha-tocotrienol quinone (Figure 1(b)) at reperfusion. Fig 1(c) shows a volumetric comparison of total infarct with administration of vehicle versus alpha-tocotrienol quinone at reperfusion.

### Example 3:

### Human Subject Trial

This trial can be designed as a randomized, double-blind, placebo-controlled efficacy study of the therapy described herein in patients presenting with acute ischemic stroke. Patients are selected for the trial based on a set of eligibility criteria that can be determined for each study. For example, the criteria can include: age (e.g., 18-85 years), focal neurological deficit lasting at least 60 minutes, CT (or MRI) compatible with clinical diagnosis of acute ischemic stroke, and NIH Stroke Scale of at least 8. The exclusion criteria can include, e.g., severe coexisting systemic disease, preexisting medical conditions that may interfere with participation, and surgery that is required within 24 hours. The protocol for the study should be approved by the institutional review board of the institution where the trial is taking place and all patients or their legal representatives should sign an informed consent.

The primary objective of this study is to determine the effects on recovery of therapy administered orally over a 6-week treatment period and a 6-week follow-up period in patients with acute ischemic stroke. The following parameters can be measured in order to evaluate recovery. Stroke lesion volume can be assessed using conventional T2-weighed MRI for all patients in the trial. Additionally, diffusion-weighted imaging (DWI) can be conducted to compare changes in lesion volume at baseline with the volume at week 12.

To be eligible for this study, the patient has to present within 24 hours with symptoms on clinical examination consistent with an acute ischemic stroke referable to the middle cerebral artery territory. Furthermore, patients must have at least 8 points on the NIHSS with at least two of these points from the motor sections.

A baseline CT or conventional MRI scan is performed to confirm that it is consistent with a diagnosis of ischemic stroke.

All patients who qualify according to the inclusion and exclusion criteria and for whom informed consent is obtained are randomly allocated on a one-to-one basis to 6 weeks of treatment with either placebo or combination therapy. Both combination therapy and placebo can be administered orally, either once or twice a day. It should be noted that other routes of administration and other dose schedules may be used. If a patient is unable to swallow, a gastric tube is placed for delivery of the drug.

Patients are seen by study personnel at baseline, 1 week, hospital discharge, 3 weeks, 6 weeks, and 12 weeks, at which time a side effect profile and drug efficacy are measured.

The efficacy of the therapy may be measured in several ways. The primary outcome measure may be, e.g., a comparison of proportion of patients in the placebo and therapy groups who have improved from baseline on their NIHSS total score by at least 7 points at week 12. Additional measures can include, e.g., the percent of patients who improve by 1 or 2 points on the Clinician's Global Impressions (CGI) scale (see Guy W., Early clinical drug evaluation unit (ECDEU) assessment manual for psychopharmacology, 1976, 217-222) at 12 weeks, the percent of patients who have at least 2-point improvement on the CGI severity scale, and assessment of mortality. As mentioned previously, DWI can be used to evaluate the changes in lesion volume at baseline and at week 12.

### Example 4

### PET Procedure

⁶²Cu is eluted from a ⁶²Zn/⁶²Cu positron generator and ⁶²Cu-ATSM is obtained by simple mixing of generator eluate (⁶²Cu-glycine) and ATSM synthesized by a previously reported method (Fujibayashi et al., (1997) Copper-62 ATSM: a new hypoxia imaging agent with high membrane permeability low redox potential. J. Nucl. Med. 38, 1155-1160). A 20-min dynamic PET scan is performed with bolus injection of ⁶²Cu-ATSM via the antecubital vein in approximatively 555 MBq with frame durations of 10s x 12, 60s x 8 and 10 min x 1. Early and delayed images are calculated using the first 3 min of PET data and the last frame of the dynamic data. ⁶²Cu-ATSM accumulation in the early phase reflects coronary blood flow (Fujibayashi et al., (1997), J. Nucl. Med.(1997) 38 (7) 1155-1160); Lewis et al., (2001) J. Nucl. Med. 42, 655-661; Obata et al., (2001) Ann. Nucl. Med. 15, 499-504; and Dearling et al., (2002) J. Biol. Inorg. Chem 7, 249-259). For ¹⁸FDG-PET, approximately 150 MBq or tracer is administered about 1 h after the ⁶²Cu-ATSM injection. Fifty minutes after the tracer injection, 10 min-PET acquisition is started.

The reconstructed images are then converted to semi-quantitative images corrected by the injection dose and subject's body weight (=standardized uptake value: SUV) for data analysis. Multiple circular regions of interest 1 cm in diameter are placed on each lesion and cerebellum, and then the mean SUV values are calculated. SUV ratios are derived from the ratio of SUV of each lesion to the SUV of the cerebellum.

Although the foregoing invention has been described in some detail by way of illustration and example purposes of clarity of understanding, it is apparent to those skilled in the art that certain minor changes and modifications will be practiced.

## Claims

1. A composition for use in treating, preventing, and/or ameliorating neuronal damage associated with a cerebral ischemic event or hypoxia in a mammalian subject in need of such treatment, said composition comprising an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof;
with the proviso that said subject does not suffer from a mitochondrial disease.

2. A composition as claimed in claim 1, for use as defined in said claim, wherein the composition comprises an effective amount of alpha-tocotrienol quinone or any stereoisomer, or mixture of stereoisomers, thereof.

3. A composition as claimed in claim 1, for use as defined in said claim, wherein the cerebral ischemic event is secondary to an occlusion of the cerebral vasculature.

4. A composition as claimed in claim 3, for use as defined in said claim, wherein the occlusion is due to a thromboembolus.

5. A composition as claimed in claim 1, for use as defined in said claim, wherein the cerebral ischemia is due to a spasm of the coronary vasculature.

6. A composition as claimed in claim 1, for use as defined in said claim, wherein the cerebral ischemic event is secondary to a cessation of cardiac function, a cardiopulmonary bypass procedure, or a hemorrhagic event in the cerebral vasculature.

7. A composition as claimed in claim 2, for use as defined in said claim, wherein said tocotrienol quinone composition comprises at least about 65% alpha-tocotrienol quinone.

8. A composition as claimed in claim 1, for use as defined in said claim, wherein said composition additionally comprises a pharmaceutically acceptable carrier.

9. A composition as claimed in claim 1, for use as defined in said claim, wherein said composition is administered orally.

10. A composition as claimed in claim 1, for use as defined in said claim, wherein said composition is administered parenterally.

11. A composition as claimed in claim 2, for use as defined in said claim, wherein said composition comprises alpha-tocotrienol quinone in a range of about 1 to about 1000 mg per kg body weight of said mammalian subject.

12. A composition as claimed in claim 2, for use as defined in said claim, wherein said composition comprises alpha-tocotrienol quinone in a range of about 1 to about 50 mg per kg body weight of said mammalian subject.

13. A composition as claimed in claim 1, for use as defined in said claim, wherein said neuronal damage is selected from neuronal cell death, total cerebral infarct volume, cerebral ischemic damage, cerebral tissue edema, and cognitive dysfunction.

14. A composition for use in treating and/or ameliorating a cerebral ischemic event or hypoxia in a mammalian subject in need of such treatment, said composition comprising an effective amount of a tocotrienol quinone selected from the group consisting of alpha-tocotrienol quinone, beta-tocotrienol quinone, gamma-tocotrienol quinone and delta-tocotrienol quinone, or a mixture thereof, or a single stereoisomer or mixture of stereoisomers thereof;
with the proviso that said subject does not suffer from a mitochondrial disease where stroke is one of the symptoms of the mitochondrial disease.

15. A composition as claimed in claim 14, for use as defined in said claim, wherein the composition comprises an effective amount of alpha-tocotrienol quinone or any stereoisomer, or mixture of stereoisomers, thereof.

16. The composition as claimed in any one of claims 1-15, for use as defined in said claim, wherein the alpha-tocotrienol quinone or stereoisomer, or mixture of stereoisomers, thereof, is the sole active ingredient present in an effective amount in the composition.

## Patentansprüche

1. Zusammensetzung zur Verwendung bei der Behandlung, Prävention und/oder Verbesserung von neuronalem Schaden verbunden mit einem zerebralen ischämischen Ereignis oder Hypoxie in einem Säugersubjekt, das einer solchen Behandlung bedarf, wobei die Zusammensetzung eine wirksame Menge von einem Tocotrienolchinon umfasst, ausgewählt aus der Gruppe bestehend aus alpha-Tocotrienolchinon, beta-Tocotrienolchinon, gamma-Tocotrienolchinon und delta-Tocotrienolchinon oder einem Gemisch davon oder einem einzigen Stereoisomer oder einem Gemisch aus Stereoisomeren davon;
mit der Maßgabe, dass das Subjekt nicht unter einer mitochondrialen Erkrankung leidet.

2. Zusammensetzung nach Anspruch 1 zur Verwendung wie in dem Anspruch definiert, wobei die Zusammensetzung eine wirksame Menge von alpha-Tocotrienolchinon oder einem beliebigen Stereoisomer oder einem Gemisch aus Stereoisomeren davon umfasst.

3. Zusammensetzung nach Anspruch 1 zur Verwendung wie in dem Anspruch definiert, wobei das zerebrale ischämische Ereignis infolge einer Okklusion des zerebralen Gefäßsystems auftritt.

4. Zusammensetzung nach Anspruch 3 zur Verwendung wie in dem Anspruch definiert, wobei die Okklusion aufgrund einer Thromboembolie auftritt.

5. Zusammensetzung nach Anspruch 1 zur Verwendung wie in dem Anspruch definiert, wobei die zerebrale Ischämie aufgrund eines Krampfes des Koronargefäßsystems auftritt.

6. Zusammensetzung nach Anspruch 1 zur Verwendung wie in dem Anspruch definiert, wobei das zerebrale ischämische Ereignis infolge einer Einstellung der Herzfunktion, einer kardiopulmonalen Bypass-Operation oder eines hämorrhagischen Ereignisses in dem zerebralen Gefäßsystem eintritt.

7. Zusammensetzung nach Anspruch 2 zur Verwendung wie in dem Anspruch definiert, wobei die Tocotrienolchinon-Zusammensetzung mindestens ungefähr 65 % alpha-Tocotrienolchinon umfasst.

8. Zusammensetzung nach Anspruch 1 zur Verwendung wie in dem Anspruch definiert, wobei die Zusammensetzung zusätzlich einen pharmazeutisch annehmbaren Träger umfasst.

9. Zusammensetzung nach Anspruch 1 zur Verwendung wie in dem Anspruch definiert, wobei die Zusammensetzung oral verabreicht wird.

10. Zusammensetzung nach Anspruch 1 zur Verwendung wie in dem Anspruch definiert, wobei die Zusammensetzung parenteral verabreicht wird.

11. Zusammensetzung nach Anspruch 2 zur Verwendung wie in dem Anspruch definiert, wobei die Zusammensetzung alpha-Tocotrienolchinon in einem Bereich von ungefähr 1 bis ungefähr 1000 mg pro kg Körpergewicht des Säugersubjekts umfasst.

12. Zusammensetzung nach Anspruch 2 zur Verwendung wie in dem Anspruch definiert, wobei die Zusammensetzung alpha-Tocotrienolchinon in einem Bereich von ungefähr 1 bis ungefähr 50 mg pro kg Körpergewicht des Säugersubjekts umfasst.

13. Zusammensetzung nach Anspruch 1 zur Verwendung wie in dem Anspruch definiert, wobei der neuronale Schaden ausgewählt ist aus neuronalem Zelltod, Hirninfarkt-Gesamtvolumen, zerebralem ischämischem Schaden, Hirngewebeödem und kognitiver Dysfunktion.

14. Zusammensetzung zur Verwendung bei der Behandlung und/oder Verbesserung eines zerebralen ischämischen Ereignisses oder Hypoxie in einem Säugersubjekt, das einer solchen Behandlung bedarf, wobei die Zusammensetzung eine wirksame Menge von einem Tocotrienolchinon umfasst, ausgewählt aus der Gruppe bestehend aus alpha-Tocotrienolchinon, beta-Tocotrienolchinon, gamma-Tocotrienolchinon und delta-Tocotrienolchinon oder einem Gemisch davon oder einem einzigen Stereoisomer oder einem Gemisch aus Stereoisomeren davon;
mit der Maßgabe, dass das Subjekt nicht unter einer mitochondrialen Erkrankung leidet, bei der ein Schlaganfall eines der Symptome der mitochondrialen Erkrankung ist.

15. Zusammensetzung nach Anspruch 14 zur Verwendung wie in dem Anspruch definiert, wobei die Zusammensetzung eine wirksame Menge von alpha-Tocotrienolchinon oder einem beliebigen Stereoisomer oder einem Gemisch aus Stereoisomeren davon umfasst.

16. Zusammensetzung nach einem der Ansprüche 1-15 zur Verwendung wie in dem Anspruch definiert, wobei das alpha-Tocotrienolchinon oder Stereoisomer oder das Gemisch aus Stereoisomeren davon der einzige Wirkstoff ist, der in der Zusammensetzung in einer wirksamen Menge vorhanden ist.

## Revendications

1. Composition pour l'utilisation dans le traitement, la prévention, et/ou l'amélioration de lésion neuronale associée à un événement ischémique cérébral ou une hypoxie chez un sujet mammifère ayant besoin d'un tel traitement, ladite composition comprenant une quantité efficace d'une tocotriénol quinone sélectionnée parmi le groupe constitué d'alpha-tocotriénol quinone, de bêta-tocotriénol quinone, de gamma-tocotriénol quinone et de delta-tocotriénol quinone, ou d'un mélange de celles-ci, ou d'un stéréoisomère unique ou d'un mélange de stéréoisomères de celles-ci ;
à condition que ledit sujet ne souffre pas de maladie mitochondriale.

2. Composition selon la revendication 1, pour l'utilisation selon ladite revendication, dans laquelle la composition comprend une quantité efficace d'alpha-tocotriénol quinone ou d'un quelconque stéréoisomère, ou d'un mélange de stéréoisomères, de celle-ci.

3. Composition selon la revendication 1, pour l'utilisation selon ladite revendication, dans laquelle l'événement ischémique cérébral est secondaire à une occlusion de la vasculature cérébrale.

4. Composition selon la revendication 3, pour l'utilisation selon ladite revendication, dans laquelle l'occlusion est due à une thromboembolie.

5. Composition selon la revendication 1, pour l'utilisation selon ladite revendication, dans laquelle l'ischémie cérébrale est due à un spasme de la vasculature coronaire.

6. Composition selon la revendication 1, pour l'utilisation selon ladite revendication, dans laquelle l'événement ischémique cérébral est secondaire à une cessation de fonction cardiaque, une procédure de pontage cardiopulmonaire, ou un événement hémorragique dans la vasculature cérébrale.

7. Composition selon la revendication 2, pour l'utilisation selon ladite revendication, dans laquelle ladite composition de tocotriénol quinone comprend au moins environ 65 % d'alpha-tocotriénol quinone.

8. Composition selon la revendication 1, pour l'utilisation selon ladite revendication, dans laquelle ladite composition comprend en outre un vecteur pharmaceutiquement acceptable.

9. Composition selon la revendication 1, pour l'utilisation selon ladite revendication, dans laquelle ladite composition est administrée oralement.

10. Composition selon la revendication 1, pour l'utilisation selon ladite revendication, dans laquelle ladite composition est administrée parentéralement.

11. Composition selon la revendication 2, pour l'utilisation selon ladite revendication, dans laquelle ladite composition comprend l'alpha-tocotriénol quinone dans une plage d'environ 1 à environ 1000 mg par kg de poids corporel dudit sujet mammifère.

12. Composition selon la revendication 2, pour l'utilisation selon ladite revendication, dans laquelle ladite composition comprend l'alpha-tocotriénol quinone dans une plage d'environ 1 à environ 50 mg par kg de poids corporel dudit sujet mammifère.

13. Composition selon la revendication 1, pour l'utilisation selon ladite revendication, dans laquelle ladite lésion neuronale est sélectionnée parmi une apoptose neuronale, un volume total d'infarctus cérébral, une lésion ischémique cérébrale, un oedème du tissu cérébral, et une dysfonction cognitive.

14. Composition pour l'utilisation dans le traitement et/ou l'amélioration d'un événement ischémique cérébral ou une hypoxie chez un sujet mammifère ayant besoin d'un tel traitement, ladite composition comprenant une quantité efficace d'une tocotriénol quinone sélectionnée parmi le groupe constitué d'alpha-tocotriénol quinone, de bêta-tocotriénol quinone, de gamma-tocotriénol quinone et de delta-tocotriénol quinone, ou d'un mélange de celles-ci, ou d'un stéréoisomère unique ou d'un mélange de stéréoisomères de celles-ci ;
à condition que ledit sujet ne souffre pas de maladie mitochondriale où un accident vasculaire cérébral est l'un des symptômes de la maladie mitochondriale.

15. Composition selon la revendication 14, pour l'utilisation selon ladite revendication, dans laquelle la composition comprend une quantité efficace d'alpha-tocotriénol quinone ou d'un quelconque stéréoisomère, ou d'un mélange de stéréoisomères, de celle-ci.

16. Composition selon l'une quelconque des revendications 1 à 15, pour l'utilisation selon ladite revendication, dans laquelle l'alpha-tocotriénol quinone ou le stéréoisomère, ou le mélange de stéréoisomères, de celle-ci, est le seul ingrédient actif présent en une quantité efficace dans la composition.
